(19) **Europäisches Patentamt**
**European Patent Office**
**Office européen des brevets**

(11) **EP 4 406 574 A1**

(12) **EUROPEAN PATENT APPLICATION**
published in accordance with Art. 153(4) EPC

(43) Date of publication:
**31.07.2024 Bulletin 2024/31**

(21) Application number: **22871549.6**

(22) Date of filing: **05.07.2022**

(51) International Patent Classification (IPC):
***A61M 16/00*** *(2006.01)*   ***A61B 5/08*** *(2006.01)*

(52) Cooperative Patent Classification (CPC):
**A61B 5/08; A61M 16/00**

(86) International application number:
**PCT/CN2022/103904**

(87) International publication number:
**WO 2023/045488 (30.03.2023 Gazette 2023/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.09.2021 PCT/CN2021/120320**

(71) Applicant: **Shenzhen Mindray Bio-Medical
Electronics Co., Ltd.
Shenzhen, Guangdong 518057 (CN)**

(72) Inventors:
• **LIU, Jinglei
  Shenzhen, Guangdong 518057 (CN)**
• **ZOU, Xinru
  Shenzhen, Guangdong 518057 (CN)**
• **MA, Zhenyuan
  Shenzhen, Guangdong 518057 (CN)**
• **ZHOU, Xiaoyong
  Shenzhen, Guangdong 518057 (CN)**

(74) Representative: **KIPA AB
Drottninggatan 11
252 21 Helsingborg (SE)**

(54) **MEDICAL DEVICE AND METHOD FOR MEASURING POSITIVE END-EXPIRATORY PRESSURE**

(57)    A medical device and a method for measuring a positive end-expiratory pressure. The method comprises: acquiring an airway pressure and an air flow rate when a medical ventilation device ventilates a patient (1); obtaining a ventilation volume according to the air flow rate (2); and then obtaining, by means of calculation, a total positive end-expiratory pressure according to a preset respiratory mechanics equation, the airway pressure, the air flow rate and the ventilation volume (3). It can be seen that no expiratory hold operation is required during the process of measuring a positive end-expiratory pressure, a normal ventilation process is not interrupted, and the method is suitable for use in both invasive and non-invasive ventilation.

FIG. 8

EP 4 406 574 A1

**Description**

TECHNICAL FIELD

The disclosure relates to a medical field, and more particularly to a medical device and a method for measuring positive end-expiratory pressure.

BACKGROUND

During normal respiratory process, lungs are in a relaxed state at an expiratory end. At this time, a pressure inside and outside a respiratory system is balanced, that is, a pressure inside alveoli is equal to a pressure at an airway opening. However, a patient with chronic obstructive pulmonary disease (COPD) etc., has a problem of restricted expiratory gas flow due to airway occlusion during an expiratory phase, leading to early termination of expiratory gas flow, which in turn increases end-expiratory lung volume, that is, a pressure inside alveoli is greater than a pressure at an airway opening at an expiratory end, resulting in endogenous PEEP (that is, PEEPi). If an expiratory time is too short during mechanical ventilation, incomplete expiration of the patient also produces endogenous PEEP (positive end-expiratory pressure). If endogenous PEEP exists, when the patient inhales spontaneously, the endogenous PEEP must first be overcome, and then an inspiratory gas flow can be generated after enabling the pressure in lungs to be lower than the pressure at the airway opening. This increases inspiratory work of the patient, and may easily cause ineffective triggering (when the inspiratory efforts of the patient cannot overcome the endogenous PEEP).

The main type of patient faced during non-invasive ventilation is COPD patient, and COPD patient is also a patient who often develops PEEPi in clinical practice. However, since non-invasive ventilation involves an open airway, it is not possible to clinically measure PEEPi by using a respiratory hold method. There is currently no existing method for measuring PEEPi during non-invasive ventilation. In addition, during invasive ventilation, expiratory hold (closing inhalation valve(s) and expiration valve(s), closing airway) is generally performed manually to balance an intrapulmonary pressure and an airway pressure, so as to measure endogenous PEEP. However, this operation interrupts normal ventilation of the patient and often cannot measure accurately, when the patient has spontaneous respiration.

SUMMARY

This disclosure mainly provides a medical device and a method for measuring positive end-expiratory pressure, which are capable of being applied for measuring positive end-expiratory pressure during invasive and non-invasive ventilation without requiring expiratory hold.

In an embodiment, a method for measuring positive end-expiratory pressure is provided, including:

obtaining an airway pressure and a gas flow rate, when a medical ventilation device ventilates a patient;
obtaining a ventilation volume according to the gas flow rate;
obtaining a total positive end-expiratory pressure, according to a preset respiratory mechanics equation, as well as the airway pressure, the gas flow rate, and the ventilation volume; wherein the respiratory mechanics equation is at least constructed from an airway pressure, a gas flow rate, a ventilation volume, and a total positive end-expiratory pressure.

In an embodiment, another method for measuring endogenous positive end-expiratory pressure is provided, including:

obtaining esophageal pressure data of a patient, when a medical ventilation device ventilates the patient; and
obtaining gas flow rate data, airway pressure data, concentration data of carbon dioxide or chest impedance data, of the patient, when a medical ventilation device ventilates the patient;
obtaining a characteristic quantity, which characterizes that the patient overcomes an endogenous positive end-expiratory pressure, according to the gas flow rate data, the airway pressure data, the concentration data of carbon dioxide, or the chest impedance data; and
obtaining the endogenous positive end-expiratory pressure according to the esophageal pressure data and the characteristic quantity.

In an embodiment, a medical device is provided, including:

a pressure sensor, which is configured to obtain an airway pressure of a patient during a ventilation process;
a flow sensor, which is configured to obtain a gas flow rate of the patient during a ventilation process;

a processor, which is configured to: obtain the airway pressure of the patient from the pressure sensor; obtain the gas flow rate of the patient from the flow sensor; obtain a ventilation volume according to the gas flow rate; and calculate to obtain a total positive end-expiratory pressure, according to a preset respiratory mechanics equation, as well as the airway pressure, the gas flow rate, and the ventilation volume; wherein the respiratory mechanics equation is at least constructed from an airway pressure, a gas flow rate, a ventilation volume, and a total positive end-expiratory pressure.

In an embodiment, another medical device is provided, including a processor, wherein the medical device further includes a pressure sensor, a flow sensor, a carbon dioxide sensor, or an impedance sensor;

the pressure sensor is configured to obtain an airway pressure of a patient during a ventilation process;
the flow sensor is configured to obtain a gas flow rate of the patient during the ventilation process;
the carbon dioxide sensor is configured to obtain a carbon dioxide concentration of the patient during the ventilation process;
the impedance sensor is configured to obtain chest impedance of the patient during the ventilation process;
obtain esophageal pressure data of the patient, when a respiratory support system ventilates the patient; and obtain gas flow rate data, airway pressure data, concentration data of carbon dioxide, or chest impedance data, of the patient, when a respiratory support system ventilates the patient;
obtain a characteristic quantity, which characterizes that the patient overcomes an endogenous positive end-expiratory pressure, according to the gas flow rate data, the airway pressure data, the concentration data of carbon dioxide, or the chest impedance data; and
obtain an endogenous positive end-expiratory pressure according to the esophageal pressure data and the characteristic quantity.

In an embodiment, another medical device is provided, including:

a memory, which is configured to store program(s); and
a processor, which is configured to implement the method as described above by executing program(s) which is(are) stored in the memory.

In an embodiment, a computer-readable storage medium is provided, including program(s), which is(are) capable of being executed, so as to implement the method as described above.

According to the medical device and the method for measuring positive end-expiratory pressure in the above embodiments, the airway pressure and the gas flow rate are obtained, when the medical ventilation device ventilates the patient; the ventilation volume is obtained according to the gas flow rate, and then the total positive end-expiratory pressure is obtained according to a preset respiratory mechanics equation, as well as the airway pressure, the gas flow rate and the ventilation volume. It can be seen that the measurement process of positive end-expiratory pressure does not require an expiratory hold operation, does not interrupt a normal ventilation process, and is suitable for use with both invasive and non-invasive ventilation.

BRIEF DESCRIPTION OF THE DRAWINGS

FIG. 1 is a structural diagram of an embodiment of a medical device provided by this disclosure.
FIG. 2 is a structural diagram of an embodiment of a medical device provided by this disclosure.
FIG. 3 is a structural diagram of an embodiment of a medical device provided by this disclosure.
FIG. 4 is a structural diagram of an embodiment of a medical device provided by this disclosure.
FIG. 5 is a structural diagram of an embodiment of a medical device provided by this disclosure.
FIG. 6 is a structural diagram of an embodiment of a medical device provided by this disclosure.
FIG. 7 is a structural diagram of an embodiment of a medical device provided by this disclosure.
FIG. 8 is a flow chart of an embodiment of a method for measuring positive end-expiratory pressure provided by this disclosure.
FIG. 9 is a flow chart of an embodiment of a method for measuring positive end-expiratory pressure provided by this disclosure.
FIG. 10 is a waveform graph of an airway pressure, a pressure generated by respiratory muscle(s), a gas flow rate, and a ventilation volume in the method shown in FIG. 8.
FIG. 11 is a waveform graph of an airway pressure, an esophageal pressure, a gas flow rate, and a carbon dioxide concentration in the method shown in FIG. 9.

## DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in the description may be combined in any appropriate manner to form various embodiments. At the same time, the steps or actions in the method description can also be exchanged or adjusted sequentially in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc. in the description are operable to distinguish different objects, rather than to describe a specific order. In addition, the terms "connection", and "linkage" mentioned in this disclosure include direct and indirect connection (linkage), unless otherwise specified.

The medical device provided by this disclosure can monitor positive end-expiratory pressure in real time during a ventilation process, does not require an expiratory hold operation, does not interrupt a normal ventilation process, and is suitable for use in both invasive and non-invasive ventilation. There are two specific implementation principles, which are explained one by one below.

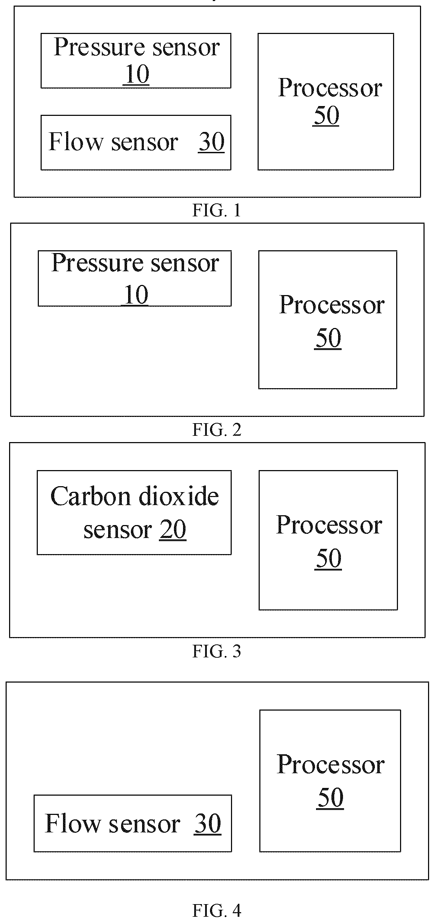

Some embodiments of this disclosure disclose a medical device. Please refer to FIGS. 1 to 4. The medical device includes a processor 50, and may further include at least one of a pressure sensor 10, a flow sensor 30, a carbon dioxide sensor 20, and an impedance sensor.

The pressure sensor 10 is configured to obtain an airway pressure of a patient during a ventilation process.

The flow sensor 30 is configured to obtain a gas flow rate of the patient during a ventilation process.

The carbon dioxide sensor 20 is configured to obtain a carbon dioxide concentration of the patient during a ventilation process.

The impedance sensor is configured to obtain chest impedance of the patient during a ventilation process.

The medical device of this disclosure can be used in various situations. For example, in some embodiments, the medical device of this disclosure can be a monitor or monitoring module, and in some embodiments, the medical device of this disclosure can be a medical ventilation device, such as a ventilator, an anesthesia machine, etc., in some embodiments, the medical device of this disclosure can be other medical device with computing and/or processing capabilities, which are described separately below.

In some embodiments, the medical device may be a monitor.

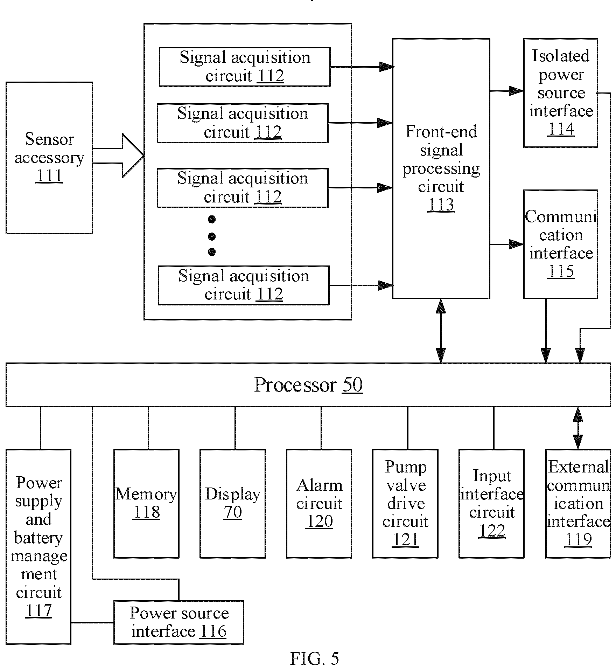

Referring FIG. 5, in some embodiments, the medical device may have an independent housing. A housing panel may have a sensor interface zone, in which multiple sensor interfaces are integrated for connecting with each external sensor accessory 111 for physiological parameter(s). The housing panel further includes one or more of a small IXD display zone, a display 70, an input interface circuit 122, and an alarm circuit 120 (such as an LED alarm zone), etc. The medical device has an external communication interface 119 and a power source interface 116 for communicating with a medical device host (such as a patient monitor, ventilator, and anesthesia machine, and so on) and taking power from the medical device host. An airway pressure and a gas flow rate are usually obtained by sensor(s) in the ventilator/anesthesia machine, so that the medical device can obtain the airway pressure and the gas flow rate of the patient during a ventilation process by communicating with the ventilator/anesthesia machine. The medical device can be arranged with a carbon dioxide sensor or an impedance sensor, which can obtain carbon dioxide concentration or chest impedance of the patient during a ventilation process. Of course, the carbon dioxide sensor or the impedance sensor can also be arranged in other devices (such as a ventilator), and the medical device obtains carbon dioxide concentration or chest impedance by communicating with other devices. The medical device also supports a build-out parameter module, can form a plug-in monitor host by means of inserting the parameter module, can be used as part of the monitor, or can be connected with the host via a cable, with the build-out parameter module being used as an external accessory of the monitor. Internal circuit(s) of the medical device is(are) disposed inside the housing. The internal circuit includes a signal acquisition circuit 112, a front-end signal processing circuit 113, which are corresponding to one or more physiological parameters, wherein the signal acquisition circuit 112 may be selected from an electrocardiogram circuit, a respiration circuit, a body temperature circuit, a blood oxygen circuit, a non-invasive blood pressure circuit, an invasive blood pressure circuit, etc. Theses signal acquisition circuits 112 are respectively electrically connected with a corresponding sensor interface, so as to be connected with the sensor accessories 111 which are corresponding to different physiological parameters, with an output end thereof being coupled to the front-end signal processing circuit 113. A communication port of the front-end signal processing circuit 113 is coupled to a processor 50, and the processor 50 is electrically connected with the external communication interface 119 and the power source interface 116. The sensor accessories 111 and the signal acquisition circuits 112, which are corresponding to different physiological parameters, may adopt common circuits in the existing technology. The front-end signal processing circuit 113 completes a sampling and analogy-to-digital conversion of an output signal of the signal acquisition circuit 112, and outputs a control signal to control measurement processes of the physiological parameters. These parameters include but are not limited to parameters of electrocardiogram, respiration, body temperature, blood oxygen saturation, non-invasive blood pressure, and invasive blood pressure. The front-end signal processing circuit 113 can be realized by a single-chip microcomputer or other semiconductor devices. The front-end signal processing circuit 113 can be powered by an isolated power source. The sampled data is simply processed and packaged, and then transmitted to the processor 50 through the isolated communication interface. For example, the front-end signal processing circuit 113 can be coupled to the processor 50 through an isolated power source interface 114 and a communication interface 115. The reason that the front-end signal processing circuit 113 is powered by an isolated power source is that the DC/DC power source isolated by a transformer plays a role in isolating the patient from the power supply device, and the main purpose is: 1. isolating the patient, in which the application part is floated above the ground through the isolation transformer such that the leakage current of the patient is small enough; and 2. preventing the voltage or energy in the application of defibrillation or electric scalpel from affecting the boards and devices of the intermediate circuit such as the main control board (guaranteed by creepage distance and electrical clearance). Of course, the front-end signal processing circuit 113 may also be connected with the processor 50 through a cable 124. The processor 50 completes the calculation of physiological parameters and transmits the calculation results and waveforms of the parameters to the host (such as a host with a display, PC, central station, etc.) through an external communication interface 119. The processor 50 is connected with the external communication interface 119 through a cable for communicating and connected with the power source interface 116 through a cable for taking power. The medical device is further provided with a power supply and battery management circuit 117, which takes power from the host through the power source interface 116 and supplies the power to the processor 50 after processing, such as rectification and filtering. The power supply and battery management circuit 117 can also monitor, manage, and protect the power obtained from the host through the power source interface 116. The external communication interface 119 may be one or a combination of local zone network interfaces composed of Ethernet, Token Ring, Token Bus, and optical fibre distributed data interface (FDDI) as the backbone of these three networks, may also be one or a combination of wireless interfaces such as infrared, Bluetooth, WIFI, and WMTS communication, or may also be one or a combination of wired data connection interfaces, such as RS232 and USB. The external communication interface 119 may also be one or a combination of the wireless data transmission interface and the wired data transmission interface. The host can be any computer device, such as a host of the monitor, and a computer, wherein a monitor can be formed by installing matching software(s) on the host. The host can also be a communication device, such as a mobile phone. The medical device transmits the data to the mobile phone supporting Bluetooth communication through a Bluetooth interface to realize a remote transmission of data. After the processor 50 completes the calculation of physiological parameters, it can also determine whether the physiological parameters are abnormal. If yes, it can alarm through the alarm circuit 120.

The above are some descriptions of a medical device as a patient monitor.

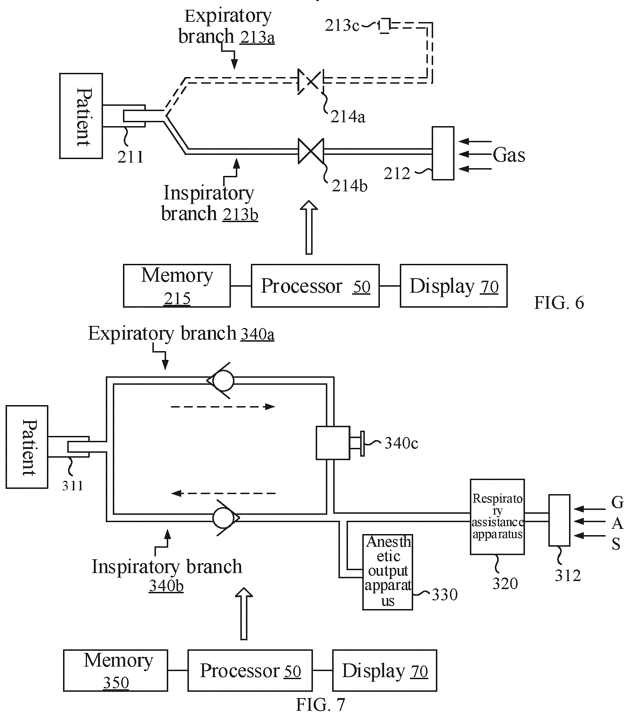

In some embodiments, the medical device may also be a ventilator. The ventilator is an artificial mechanical ventilation device used to assist or control a spontaneous respiratory movement of a patient, so as to achieve gas exchange in lungs, reduce a consumption of human body, and facilitate restoration of respiratory function. Please refer to FIG. 6, which shows an invasive ventilator. In some embodiments, the medical device may also include a respiratory support system and a display 70. The respiratory support system is configured to ventilate the patient to provide respiratory support. The respiratory support system may include: a respiratory interface 211, a gas source interface 212, a respiratory loop and a respiratory assistance apparatus.

The respiratory loop selectively connects the gas source interface 212 with a respiratory system of the patient. In some embodiments, such as in an invasive ventilator, the respiratory loop includes an expiratory branch 213a and an inspiratory branch 213b. The expiratory branch 213a is connected between the respiratory interface 211 and an exhaust port 213c, and is configured to discharge an exhaled gas of the patient to the exhaust port 213c. The exhaust port 213c can be connected with external environment, or can be connected with a dedicated gas recovery apparatus. In some embodiments, such as a non-invasive ventilator, the respiratory loop includes an inspiratory branch 213b, but does not require an expiratory branch 213a. The gas source interface 212 is connected with a gas source (not shown). The gas source is configured to provide a gas, which can usually be oxygen, air, etc. In some embodiments, the gas source can be a compressed gas bottle or a central gas source, which supplies a gas to the ventilator through the gas source interface 212. The type of the supplied gas includes oxygen O2 and air. The gas source interface 212 can include conventional components, such as a pressure gauge, a pressure regulator, a flow meter, a pressure relief valve and a regulation and protection apparatus for air-to-oxygen ratio, which are configured to control flow amounts of various gases (such as oxygen and air). Of course, for an electric ventilator, the gas source can employ a turbine which outputs the gas to the gas source interface 212. The inspiratory branch 213b is connected between the respiratory interface 211 and the gas source interface 212, and is configured to provide oxygen or air to the patient. For example, the gas, which is inputted from the gas source interface 212, enters into the inspiratory branch 213b, and then enters into lungs of the patient after passing through the respiratory interface 211. The respiratory interface 211 is configured to connect the patient to the respiratory loop. In addition to introducing the gas transmitted from the inspiratory branch 213b to the patient, for an invasive ventilator, the respiratory interface 211 also introduces the gas exhaled by the patient through the expiratory branch 213a, while for a non-invasive ventilator, the respiratory interface 211 can directly discharge the gas exhaled by the patient. Depending on situations, the respiratory interface 211 can be a nasal cannula or a mask worn on mouth and nose. The respiratory assistance apparatus is connected with the gas source interface 212 and the respiratory loop, and controls to deliver a gas, which is provided by the external gas source, to the patient through the respiratory loop. In an embodiment of an invasive ventilator, the respiratory assistance apparatus may include an expiratory controller 214a and an inspiratory controller 214b. In an embodiment of the non-invasive ventilator, the respiratory assistance apparatus may include an inspiratory controller 214b. The expiratory controller 214a is arranged at the expiratory branch 213a, and is configured to open or close the expiratory branch 213a according to a control instruction, or to control a flow rate or a pressure of the exhaled gas of patient. In specific implementation, the expiratory controller 214a may include one or more of devices that can control a flow rate or pressure, such as an expiratory valve, a one-way valve, a flow controller, a PEEP valve, and others. The inspiratory controller 214b is arranged at the inspiratory branch 213b and is configured to open or close the inspiratory branch 213b according to a control instruction, or to control a flow rate or pressure of the outputted gas. In specific implementation, the inspiratory controller 214b may include one or more of devices that can control a flow rate or pressure, such as an inspiratory valve, a one-way valve, a flow controller, a PEEP valve, and others.

The memory 215 can be configured to store data or program(s), such as data obtained by a sensor, data calculated by a processor, or image frame(s) generated by a processor. The image frame(s) can be a 2D or 3D image(s), or the memory 215 can store a graphic user interface, one or more default image display settings, and programming instruction(s) for a processor. The memory 215 may be a tangible and non-transitory computer-readable medium, such as flash memory, RAM, ROM, EEPROM, etc.

In some embodiments, the processor 50 is configured to execute instruction(s) or program(s), so as to control the respiratory assistance apparatus, the gas source interface 212 and/or various control valves in the respiratory loop, or so as to process the received data to generate the required calculation or determination result, or so as to generate visual data or graphic(s) and output the visual data or graphic(s) to the display 70 for displaying.

The above are some description of a medical device as a ventilator. It should be noted that FIG. 6 above is just an example of a ventilator, and it does not limit the structure of the ventilator.

In some embodiments, the medical device may also be an anesthesia machine, which is mainly configured to provide an anesthetic gas, deliver the anesthetic gas to the respiratory system of the patient through a respirator, and control an inhaled amount of the anesthetic gas. Referring to FIG. 7, a medical device of some embodiments may also include a respiratory support system, an anesthetic output apparatus 330, a memory 350, and a display 70. The respiratory support system is configured to ventilate the patient, so as to provide respiratory support. The respiratory support system may include: a respiratory interface 311, a gas source interface 312, a respiratory assistance apparatus 320 and a respiratory loop.

The gas source interface 312 is connected with a gas source (not shown), which is configured to provide a gas. The gas can usually be oxygen, nitrous oxide (laughing gas) or air. In some embodiments, the gas source can be a compressed gas bottle or a central gas supply source, which supplies a gas to the anesthesia machine through the gas source interface 312. The type of the supplied gas includes oxygen 02, laughing gas $N_2O$, air, etc. The gas source interface 312 may include conventional components, such as a pressure gauge, a pressure regulator, a flow meter, a pressure relief valve and a regulation and protection apparatus for air-to-oxygen ratio, which are configured to control flow amounts of various gases (such as oxygen and air). The gas, which is inputted from the gas source interface 312, enters into the respiratory loop, and forms a mixed gas with an original gas inside the respiratory loop.

The respiratory assistance apparatus 320 is configured to provide power for non-spontaneous respiration of the patient and maintain a smooth airway. In some embodiments, the respiratory assistance apparatus 320 is connected with the gas source interface 312 and the respiratory loop, and controls the gas, which is provided by an external gas source, to the patient through the respiratory loop. In some specific embodiments, the respiratory assistance apparatus 320 mixes a fresh gas, which is inputted from the gas source interface 312 with the gas exhaled by the patient in the respiratory loop and with an anesthetic, which is outputted from the anesthetic output apparatus 330, and then outputs the anesthetic mixture to the respiratory interface 311 through the inspiratory branch 340b, so as to drive the patient to inhale, and receives the exhaled gas of patient through the expiratory branch 340a. In specific embodiments, the respiratory assistance apparatus 320 generally includes a machine-controlled ventilation module, and a gas flow conduit of the machine-controlled ventilation module is connected with the respiratory loop. In an anesthesia maintenance stage during surgery or when the patient has not recovered spontaneous respiration, the machine-controlled ventilation module is configured to provide the patient with respiratory power. In some embodiments, the respiratory assistance apparatus 320 further includes a manual ventilation module, and a gas flow conduit of the manual ventilation module is connected with the respiratory loop. During an induction phase before the patient is intubated during surgery, a manual ventilation module is often required to assist the respiration of the patient. When the respiratory assistance apparatus 320 includes both a machine-controlled ventilation module and a manual ventilation module, the machine-controlled and the manual ventilation mode can be switched through a machine-controlled switch or a manual switch (such as a three-way valve), so that the machine-controlled ventilation module or the manual ventilation mode can be switched, so as to connect the machine-controlled ventilation module or the manual ventilation mode with the respiratory loop, thus controlling the respiration of the patient. Those skilled in the art should understand that the anesthesia machine may only include a machine-controlled ventilation module or a manual ventilation module according to specific requirements.

The anesthetic output apparatus 330 is configured to provide an anesthetic. Normally, the anesthetic are mixed in the form of gas into the fresh air, which is introduced by the gas source interface 312, and then delivered together to the respiratory loop. In a specific embodiment, the anesthetic output apparatus 330 is implemented as an anesthetic volatilization pot. The anesthetic is usually in a liquid state and is stored in the anesthetic volatilization pot. Optionally, the anesthetic volatilization pot may include a heating device for heating the anesthetic to volatilize for generating anesthetic vapor. The anesthetic output apparatus 330 is connected with a pipeline of the gas source interface 312, the anesthetic vapor is mixed with a fresh air, which is introduced by the gas source interface 312, and then delivered together into the respiratory loop.

In some embodiments, the respiratory loop may include an inspiratory branch 340b, an expiratory branch 340a, and a soda-lime tank 340c. The inspiratory branch 340b and the expiratory branch 340a are connected, so as to form a closed loop, and the soda-lime tank 340c is arrange at a pipeline of the expiratory branch 340a. A mixed gas of fresh air, which air is introduced by the gas source interface 312, is inputted from an inlet of the inspiratory branch 340b, and is provided to the patient through the respiratory interface 311 arranged at the outlet of the inspiratory branch 340b. The respiratory interface 311 may be a mask, nasal cannula, or endotracheal tube. In a preferred embodiment, the inspiratory branch 340b is provided with a one-way valve, which is opened during an inspiratory phase and closed during an expiratory phase. The expiratory branch 340a is also provided with a one-way valve, which is closed during an inspiratory phase and opened during an expiratory phase. An inlet of the exhalation branch 340a is connected with the respiratory interface 311. When the patient exhales, the exhaled gas enters into the soda-lime tank 340c after passing through the expiratory branch 340a, and carbon dioxide in the exhaled gas is filtered by a material in the soda-lime tank 340c. The gas is recycled into the suction branch 340b after filtering carbon dioxide.

The memory 350 can be configured to store data or program(s), such as data obtained by a sensor, data calculated by a processor, or image frame(s) generated by a processor. The image frame(s) can be a 2D or 3D image(s), or the memory 350 can store a graphic user interface, one or more default image display settings, and programming instruction(s) for a processor. The memory 350 may be a tangible and non-transitory computer-readable medium, such as flash memory, RAM, ROM, EEPROM, etc.

The processor 50 is configured to execute instruction(s) or programs, so as to control the respiratory assistance apparatus 320, the gas source interface 310 and/or various control valves in the respiratory loop, or so as to process the received data to generate the required calculation or determination result, or so as to generate visual data or graphic(s) and output the visual data or graphic(s) to the display 70 for displaying.

The above are some descriptions of the medical device as an anesthesia machine. It should be noted that FIG. 7 above is just an example of an anesthesia machine, and it does not limit the structure of the anesthesia machine.

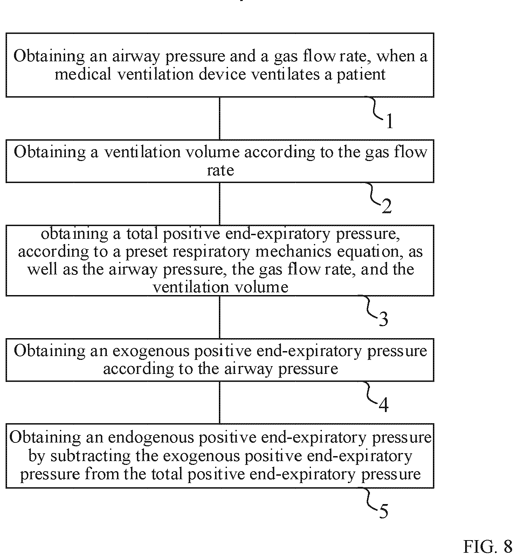

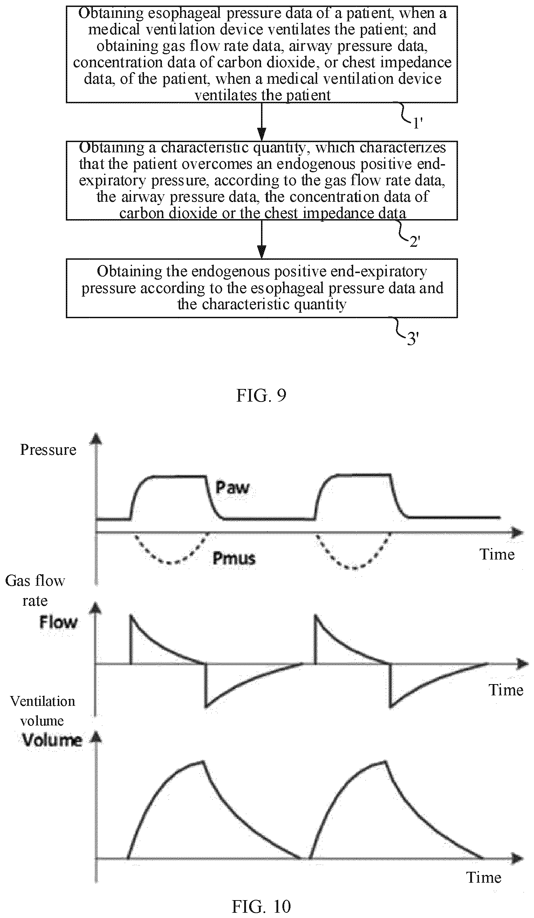

When a medical device detects a positive end-expiratory pressure, the method in FIG. 8 or the method in FIG. 9 can be used. This embodiment uses FIG. 8 as an example to illustrate, which includes the following steps.

In step 1, an airway pressure and a gas flow rate are obtained, when a medical ventilation device ventilates a patient. The medical device is a ventilator or an anesthesia machine, as shown in FIG. 1, and includes: a pressure sensor 10, a processor 20, and a flow sensor 30, and may also include hardware(s) shown in FIGS. 6 or 7. The pressure sensor 10 can be arranged at a position, where is connected with an airway of the patient, for example, the pressure sensor 10 can be arranged inside a pipeline of a medical ventilation device or at a respiratory mask. When the respiratory support system ventilates (provides respiratory support) to the patient, the pressure sensor 10 obtains an airway pressure of the patient. The flow sensor 30 may also be arranged at a position, where is connected with an airway of the patient, such as a respiratory interface or a respiratory loop. When the respiratory support system ventilates the patient, the flow sensor 30 obtains a gas flow rate during ventilation, that is, when the gas flow rate is obtained, a gas flow amount is known based on an inner diameter of pipe, and then a ventilation volume is known based on time. The processor 50 obtains the airway pressure of the patient obtained by the pressure sensor 10 and the gas flow rate obtained by the flow sensor 30. If the medical device is a monitor, the monitor is in communicative connection with the medical ventilation device, and may include the pressure sensor 10, the processor 20 and the flow sensor 30 as shown in FIG. 1, and may also include hardware(s) as shown in FIG. 5. The processor 50 of the monitor obtains an airway pressure of the patient through its own pressure sensor 10 and obtains a gas flow rate of the patient through its own flow sensor 30. Of course, in some embodiments, the pressure sensor 10 and the flow sensor 30 are not necessary for the monitor. The processor 50 of the monitor can receive an airway pressure of the patient obtained by a pressure sensor of a medical ventilation device, and can receive a gas flow rate of the patient obtained by a flow sensor of a medical ventilation device.

Since there is no need to perform an expiratory hold operation, the pressure sensor 10 can obtain the airway pressure of the patient in real time, and the flow sensor 30 can obtain the gas flow rate of ventilation in real time.

In step 2, the processor 50 obtains a ventilation volume according to the gas flow rate. For example, the processor 50 integrates the gas flow rate over time to obtain the ventilation volume, that is, a volume of a gas, which gas is outputted by the respiratory support system. The processor 50 can calculate a real-time gas flow rate to obtain a real-time ventilation volume. In a respiratory mechanics equation, for invasive ventilation which usually has good sealing, the gas flow rate, which is obtained by the sensor, can reflect an amount of a gas, which gas enters into a body of the patient; while for non-invasive ventilation which has gas leakage due to characteristics of ventilation method, the gas flow rate, which is obtained by the sensor, can be compensated to obtain an actual flow rate, and then a total positive end-tidal pressure can be calculated through the respiratory mechanics equation. Specifically, the processor 50 obtains a leakage amount of the gas during non-invasive ventilation, wherein the leakage amount of the gas may be a patient-side leakage, which is obtained by subtracting a system leakage amount from a total leakage amount.

In step 3, the processor 50 obtains a total positive end-expiratory pressure PEEPtot, according to a preset respiratory mechanics equation, as well as the airway pressure, the gas flow rate and the ventilation volume; wherein, the respiratory mechanics equation is at least constructed from an airway pressure Paw, a pressure generated by respiratory muscle(s) Pmus, a gas flow rate Flow, a ventilation volume Volume, and a total positive end-expiratory pressure PEEPtot. It can be seen from the above that, an airway pressure and a gas flow rate are obtained or calculated by the patient during normal ventilation. Therefore, the measurement process of positive end-expiratory pressure does not require an expiratory hold operation, does not interrupt a normal ventilation process, and is suitable for both invasive and Non-invasive ventilation. When the medical ventilation device ventilates the patient, it maintains a certain positive pressure in a respiratory tract of the patient at expiratory end, which can avoid early alveolar closure and allow some alveoli that have lost their ventilation function due to exudation, atelectasis and other reasons to recruit, thereby increasing the reduced functional residual capacity and achieving a purpose of improving oxygenation. Total positive end-expiratory pressure PEEPtot usually consists of two parts: exogenous positive end-expiratory pressure PEEPe and endogenous positive end-expiratory pressure PEEPi. Exogenous positive end-expiratory pressure PEEPe, as the name suggests, is a positive pressure in a respiratory tract of a patient at expiratory end, which pressure is caused by external cause(s), and is usually generated, when a medical ventilation device ventilates the patient. The user can adjust the exogenous positive end-expiratory pressure PEEPe by setting up the medical ventilation device. Endogenous positive end-expiratory pressure (PEEPi) is a positive pressure in a respiratory tract of the patient at expiratory end, which pressure is caused by internal cause(s), such as lung condition of the patient. The exogenous positive end-expiratory pressure PEEPe and the endogenous positive end-expiratory pressure PEEPi finally add up to form the total positive end-expiratory pressure PEEPtot.

The processor 50 can display the total positive end-expiratory pressure PEEPtot through a connected display 70 for easy viewing by a doctor. Since the airway pressure and the gas flow rate can be obtained in real time by corresponding sensors, the processor 50 can calculate the ventilation volume in real time, and then calculate the total positive end expiratory pressure PEEPtot in real time. Therefore, the total positive end expiratory pressure PEEPtot displayed on the display 70 can be in real time, it is helpful for doctor to grasp a ventilation state of the patient, to accurately control respiration, anesthesia, etc., and to improve a safety of patient ventilation.

The respiratory system can be simplified as a first-order RC system, where R represents a resistance of respiratory system (a viscous resistance of respiratory system), and C represents compliance of respiratory system (can also be represented by an elastic resistance of respiratory system E). During mechanical ventilation, a respiratory driving force mainly comes from two aspects. One is a driving force of the ventilator or anesthesia machine minus an airway pressure (Paw), and the other is a contraction force of patient for a ventilator (Pmus). According to the first-order model, the respiratory mechanics equation can be obtained as:

$$PEEPtot = k4*Paw + k1*Pmus - k2*Flow*R - k3*Volume*E.$$

Paw represents the airway pressure, which can be directly measured by an airway pressure sensor during mechanical ventilation.

Flow represents the gas flow rate, which can be measured by a flow sensor.

Volume represents the ventilation volume, which is an integral of the gas flow rate.

k1, k2, k3 and k4 represent preset empirical coefficients, wherein each empirical coefficient can be the same or different, and can be built into system or set by a user (such as medical staff). In an embodiment, k1, k2, k3 and k4 may all be 1.

PEEPtot represents the total positive end-expiratory pressure (i.e., total PEEP), which is equal to a sum of the exogenous positive end-expiratory pressure PEEPe (exogenous PEEP, that is, PEEP, which is monitored by an airway pressure) and the endogenous positive end-expiratory pressure PEEPi (that is, endogenous PEEP).

Pmus represents the pressure, which is generated by respiratory muscle(s) of a patient.

R represents the viscous resistance of a respiratory system of a patient, which is usually a constant;

E represents the elastic resistance of a respiratory system of a patient, which is usually a constant.

A graph of each variable in the respiratory mechanics equation is shown in FIG. 10. This step can be achieved by solving the respiratory mechanics equation. Specifically, in the respiratory mechanics equation, PEEPtot, Pmus, R, and E are unknown quantities, and the others are known quantities. During an inspiratory process of the patient, Pmus changes with time, such that a series of Paw, Flow, and Volume can be obtained through measurement, so as to obtain multiple equations:

$$PEEPtot=k4*Paw(1)+k1*Pmus(1)-k2*Flow(1)*R-k3*Volume(1)*E,$$

$$PEEPtot=k4*Paw(2)+k1*Pmus(2)-k2*Flow(2)*R-k3*Volume(2)*E,$$

$$\cdots\cdots$$

$$PEEPtot=k4*Paw(n)+k1*Pmus(n)-k2*Flow(n)*R-k3*Volume(n)*E.$$

In other words, the processor 50 substitutes multiple sets of airway pressure Paw, gas flow rate Flow and ventilation volume Volume at different times as known quantities into the preset respiratory mechanics equation, and obtains a set of equations, in which unknown quantities are the pressure, which is generated by respiratory muscle(s) of the patient Pmus, and the total positive end-expiratory pressure PEEPtot.

Taking n sets of Paw, Flow and Volume data as an example, n equations can be obtained, but there are n+3 unknown quantities. Therefore, in order to make the set of equations solvable, this disclosure creatively simplifies Pmus. For example, Pmus is simplified according to physiological characteristic(s) of spontaneous respiration into a known function (curve), in other words, in the respiratory mechanics equation, pressure, which is generated by respiratory muscle(s), is an unknown quantity that has a preset functional relationship with time. Although it is an unknown quantity, it also has rule(s) (it has a default functional relationship with time). The preset functional relationship may include: an exponential function, a trigonometric function, a piecewise function, or a polynomial function, or combination(s) thereof. According to physiological characteristics of spontaneous respiration, as shown in FIG. 10, a trend of change for an absolute value of Pmus is from small to large, and then from large to small. Therefore, an exponential function can be selected to simulate a change from small to large, and then another exponential function can be selected to simulate a change from large to small. Of course, these two exponential functions actually form a piecewise function. Similarly, a linear function can be selected to simulate a change from small to large, and then another linear function can be selected to simulate a change from large to small. Similarly, these two linear functions actually form a piecewise function. A trigonometric function, a multi-order polynomial function, etc., can also be selected to simulate. Depending on a specific functional relationship selected, corresponding numbers of unknown quantities are different. For example, a sine function (such as, $\sin(b_0+b_1 t)$), a cosine function, a quadratic polynomial function (such as, $a_1 t+a_2 t^2$), can be used to simulate Pmus, so as to simplify a number of unknown quantities to 5. This embodiment uses a parabola (quadratic polynomial function) to simplify Pmus as an example. The above equations can be simplified as:

$$Y=[Paw(1), Paw(2), \ldots, Paw(n)]^T$$

$$F=[Flow(1), Flow(2), \ldots, Flow(n)]^T$$

$$V=[Volume(1), Volume(2), \ldots, Volume(n)]^T$$

Let:

$$X=[F, V, 1, t, t^2], A=[R, E, PEEPtot, a_1, a_2]^{T}.$$

Then the above set of equations can be represented by a matrix Y=X*A. The total positive end-expiratory pressure PEEPtot can be obtained by solving the above set of equations, that is, by implementing a matrix solution. Specifically, a number of data sampling points for a general inspiratory process (a number of groups of Paw, Flow and Volume data) is much greater than 5, so the set of equations is an overdetermined set of equations, that is, a number of n satisfies a requirement to solve n+3 overdetermined equations. If R and E are known, a requirement to solve n+1 overdetermined equations is also satisfied. The optimal solution of the overdetermined equations can be solved through the following process:

As Y=X*A, then $X^T*Y=X^T*X*A$; wherein $X^T*X$ is a 5*5 matrix, and implement an inverse operation, so as to obtain:

$$A=(X^T*X)^{-1}X^T*Y.$$

Then PEEPtot can be obtained by implementing a matrix solution.

Further, in order to improve an accuracy and effectiveness of solution results, one or more constraints may be added in a solving process of the set of equations, that is, when the processor 50 calculates the total positive end-expiratory pressure PEEPtot, it further sets a pressure Pmus, which is respectively generated by respiratory muscle(s) at an inspiratory beginning time and at an inspiratory ending time, both to zero (referring waveform of Pmus in FIG. 10); and/or sets the endogenous positive end-expiratory pressure PEEPi to be greater than or equal to zero; and/or sets a pressure Pmus, which is generated by respiratory muscle(s), to zero for a patient without spontaneous respiration (of course, a corresponding empirical coefficient k1 can be set to zero); and/or limits a viscous resistance R of a respiratory system and an elastic resistance E of a respiratory system into a preset reasonable range. The preset reasonable range can be set based on actual conditions, clinical experience, etc. One or more of these four constraints can be selected as constraint(s) when solving the set of equations, so as to solve PEEPtot more accurately and reliably.

Obtaining a total positive end-expiratory pressure PEEPtot facilitates the doctor to set a positive end-expiratory pressure. For example, the processor 50 obtains a recommended value for setting a positive end-expiratory pressure of a respiratory support system of a medical ventilation device according to the total positive end-expiratory pressure PEEPtot, and then the processor 50 displays the recommended value through the display. After seeing the recommended value, the medical staff can send an instruction to set the positive end-expiratory pressure to the recommended value through an input apparatus. After receiving the instruction through the input apparatus, the processor 50 responds to the instruction and sets the recommended value as the positive end-expiratory pressure, so that the respiratory support system of the medical ventilation device ventilates the patient according to the recommended value. Of course, the recommended value can also be set automatically. For example, the processor 50 automatically sets the recommended value, so that the respiratory support system ventilates the patient according to the recommended value. The exogenous positive end-expiratory pressure PEEPe is usually generated by the medical ventilation device and needs to be set. Therefore, in this embodiment, the above recommended value is the recommended value for setting the exogenous positive end-expiratory pressure PEEPe. Typically, the recommended value is 70-80% of a total positive end-expiratory pressure PEEPtot.

In some embodiments, an endogenous positive end-expiratory pressure PEEPi can also be calculated based on a total positive end-expiratory pressure PEEPtot, as shown in FIG. 8, which can also include the following steps.

In step 4, the processor 50 obtains an exogenous positive end-expiratory pressure PEEPe according to the airway pressure Paw. An existing technology can be used to calculate the exogenous positive end-expiratory pressure PEEPe by using the airway pressure Paw, and is not given in detail in this disclosure. As mentioned above, the airway pressure Paw can be obtained in real time, and the processor can calculate a real-time exogenous positive end-expiratory pressure PEEPe, according to the real-time airway pressure Paw.

In step 5, the processor 50 subtracts the exogenous positive end-expiratory pressure PEEPe from the total positive end-expiratory pressure PEEPtot, so as to obtain an endogenous positive end-expiratory pressure PEEPi. Since both the total positive end-expiratory pressure PEEPtot and the exogenous positive end-expiratory pressure PEEPe can be calculated in real time, the endogenous positive end-expiratory pressure PEEPi can also be calculated in real time. The expiratory hold method commonly used in the prior art to measure endogenous positive end-expiratory pressure PEEPi not only requires interrupting ventilation of patient, but also cannot achieve real-time measurement. However, the above method adopted in this disclosure does not require expiratory hold, and the endogenous positive end-expiratory pressure PEEPi can be obtained in real time without being restricted by invasive or non-invasive ventilation, which is very convenient.

The processor 50 can display, through a connected display, the total positive end-expiratory pressure PEEPtot, the endogenous positive end-expiratory pressure PEEPi, or both pressures, so as to facilitate a doctor to understand a ventilation state of the patient. Since the total positive end-expiratory pressure PEEPtot and the endogenous positive end-expiratory pressure PEEPi can be obtained in real time, the total positive end-expiratory pressure PEEPtot and/or the endogenous positive end-expiratory pressure PEEPi can be displayed in real time and timely, which is helpful for a doctor to diagnose and treat the patient.

The processor 50 can also determine whether the total positive end-expiratory pressure PEEPtot exceeds a first preset value, and if so, display alarm information through the display; and can also determine whether the endogenous positive end-expiratory pressure PEEPi exceeds a second preset value, and if so, display alarm information through the display. The first preset value and the second preset value can be set as needed. In this way, the total positive end-expiratory pressure PEEPtot and an endogenous positive end-expiratory pressure PEEPi of the patient can be avoided from being too high, thereby improving the safety of ventilation.

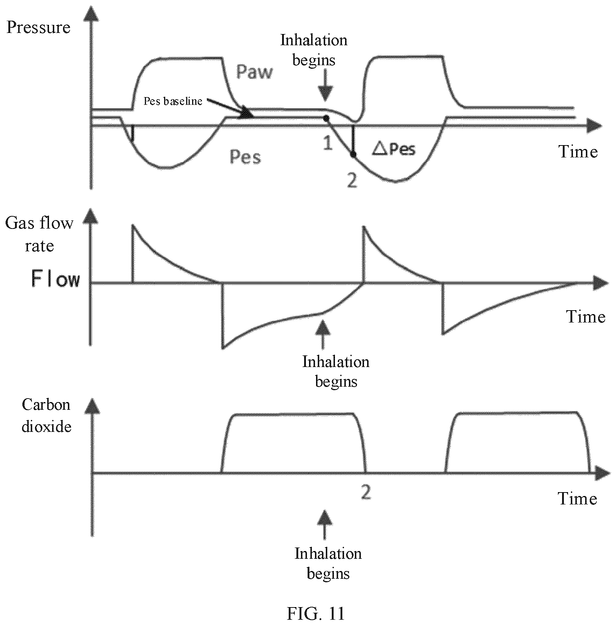

When the medical device detects a positive end-expiratory pressure, in addition to using the method in FIG. 8, the method in FIG. 9 can also be used. If an esophageal pressure Pes can be monitored in real time during mechanical ventilation, an endogenous positive end-expiratory pressure PEEPi can be calculated based on an esophageal pressure Pes. In clinical practice, an esophageal pressure is generally used to approximate an intrathoracic pressure. When the patient has an endogenous positive end-expiratory pressure PEEPi, it must first be overcome when making an inspiratory effort, so as to induce an inspiratory flow rate. As shown in FIG. 11, the esophageal pressure from point 1 to point 2 drops by $\Delta$Pes, no increase of flow rate is caused by a pressure of $\Delta$Pes, which pressure is generated by spontaneously inhalation. That is, no inspiratory flow rate is generated. Accordingly, the pressure difference of $\Delta$Pes is a pressure to overcome an endogenous positive end-expiratory pressure PEEPi, that is, PEEPi$\approx\Delta$Pes. Therefore, this method can be configured to monitor an endogenous positive end-expiratory pressure PEEPi in real time. Point 1 does not require special identification, and is a baseline value of esophageal pressure, that is, a stable value of esophageal pressure during expiration. Point 2 can be identified from flow rate data, and is a time point where a flow rate of the patient crosses zero. In addition, a time point of point 2 can also be determined through $CO_2$ waveform curve. However, there is generally a time delay in a $CO_2$ waveform curve and the time delay is a relatively certain value, therefore, the time point of point 2 can also be determined through the $CO_2$ waveform curve. FIG. 11 is a schematic diagram. For convenience of explanation, waveform curves for $CO_2$, pressure and flow rate have been aligned in time. A detailed explanation is given below with reference to FIG. 9.

The method in FIG. 9 includes the following steps.

In step 1', the processor 50 obtains esophageal pressure data of a patient, when a respiratory support system of a medical ventilation device ventilates the patient. The esophageal pressure data can be obtained by a medical device to which the processor 50 belongs, or can be obtained by the medical device to which the processor 50 belongs through communicating with and obtaining from another device (such as a medical ventilation device, a monitor, etc.). The processor 50 can also obtain target parameter data of the patient, when a respiratory support system ventilates the patient. The target parameter data can be gas flow rate data, airway pressure data, concentration data of carbon dioxide, or chest impedance data. Correspondingly, the target parameter data can be obtained by a sensor of the medical device itself. For example, the medical device includes a processor 50 and at least one of a pressure sensor 10, a flow sensor 30, a carbon dioxide sensor 20 and an impedance sensor (as shown in FIGS. 2-4). Of course, in some embodiments, the target parameter data can also be obtained by the medical device from other devices by communicating with another device (such as a medical ventilation device, a monitor, etc.).

In step 2', the processor 50 obtains a characteristic quantity, which characterizes that the patient overcomes an endogenous positive end-expiratory pressure, according to the target parameter data. The characteristic quantity can have multiple specific expression forms, such as a target time point, a target parameter which corresponds to a target time point, a target time period, a target parameter which corresponds to a target time period, etc. In this embodiment, a target time point is used as a characteristic quantity as an example. The processor 50 can identify a time point, when inhalation of the patient actually begins, as a target time point, according to gas flow rate data, airway pressure data, concentration data of carbon dioxide, or chest impedance data, such as a time point as shown in FIG. 11, when the flow rate changes from a negative value of ordinate to a positive value of ordinate (an positiveness and negativeness of a flow rate indicates a direction of the flow rate), wherein said time point corresponds to point 2, which is a time point when a gas begins to enter into a body of the patient. Although inhalation begins at point 1, due to existence of the endogenous positive end-expiratory pressure PEEPi, the gas cannot actually enter into lungs of the patient. It can be seen that the target parameter data at least includes target parameters (a gas flow rate, an airway pressure, a carbon dioxide concentration, or a chest impedance) before and after the gas flow rate changes to a positive value during the inspiratory phase. The target time point can be determined based on these target parameters. For example, the target parameter data can at least include target parameters within a period of time after a beginning of inhalation. The target parameters during this period can be data within one respiratory cycle. Since the data of patient in each respiratory cycle are basically not quite different, it can be data in different respiratory cycles. In order to improve real-time performance, the target parameter data obtained by the processor 50 may be obtained in real time by the corresponding sensor, that is, the target parameter data may be real-time data. The esophageal pressure data can at least include esophageal pressure(s) at one or more moments during an expiratory phase, and esophageal pressure(s) within a period of time after a beginning of inhalation. Similarly, esophageal pressures at different moments can come from one respiratory cycle or from different respiratory cycles. In order to improve real-time performance, the esophageal pressure data obtained by the processor 50 may be obtained in real time by the corresponding sensor, that is, the esophageal pressure data may be real-time data.

There are many specific methods for determining the target time point. For example, the processor 50 can obtain a trend of change for a gas flow rate according to the gas flow rate data (waveform curve in FIG. 11). Based on said trend of change, the time point when the gas flow rate changes to a positive value during the inhalation phase can be determined, referring a time point shown in FIG. 11, when the gas flow rate rises linearly. Of course, the target time point, when the gas flow rate changes to a positive value, can also be directly determined based on a value of the gas flow rate data, such as a time point, which corresponds to a maximum positive value of the gas flow rate.

For another example, the processor 50 obtains a minimum value of an airway pressure during the inspiratory phase according to the airway pressure data. The processor 50 can obtain a trend of change for the airway pressure Paw according to the airway pressure data (waveform curve(s) in FIG. 11) and determines the minimum value of the airway pressure during the inspiratory phase according to the trend of change for the airway pressure Paw, referring a minimum point of the waveform curve(s) of the airway pressure Paw in FIG. 11. Of course, the minimum value of the airway pressure Paw can also be directly determined based on a value of the airway pressure data. The processor 50 uses a time point which corresponds to the minimum value as the target time point.

For another example, the processor 50 can obtain a time point when a high concentration of carbon dioxide changes to a low concentration of carbon dioxide according to the concentration data of carbon dioxide, such as a trend of change for the carbon dioxide concentration (such as waveform curve(s)) in FIG. 11, and use this time point as the target time point. Specifically, a time point when the carbon dioxide concentration has a fastest decreasing rate, can be obtained according to a trend of change for the carbon dioxide concentration, and said time point when the carbon dioxide concentration has a fastest decreasing rate is used as the target time point. Of course, the processor 50 may set a time point, when the carbon dioxide concentration begins to be less than a preset concentration threshold, as the target time point, according to the concentration data of carbon dioxide, such as a trend of change for the carbon dioxide concentration in FIG. 11. The preset concentration threshold can be set as needed, such as 1/3 of a peak concentration of carbon dioxide, etc.

For another example, the processor 50 obtains chest impedance waveform(s) according to the chest impedance data, obtains a slope of each point of the chest impedance waveform(s) according to the chest impedance waveform(s), and uses a time point, when the slope begins to be greater than a preset slope threshold, as the target time point. The preset slope thresholds can be set as needed.

Of course, other methods can be configured to determine the target time point. The above methods for determining the target time point are just examples.

In step 3', the processor 50 obtains the endogenous positive end-expiratory pressure according to the esophageal pressure data and the characteristic quantity. In this embodiment, the processor 50 obtains the endogenous positive end-expiratory pressure according to the esophageal pressure data and the target time point. Specifically, the processor 50 obtains a stable value of an esophageal pressure during an expiratory phase according to the esophageal pressure data. For example, the processor 50 obtains a trend of change (such as waveform curve(s)) for the esophageal pressure according to the esophageal pressure data, and obtains the stable value of the esophageal pressure during the expiratory phase based on the trend of change for the esophageal pressure. As shown in FIG. 11, the esophageal pressure Pes basically does not change during the expiratory phase. At this time, the esophageal pressure is relatively stationary (Pes baseline in the figure). An esophageal pressure value or an average value of esophageal pressures during this period can be taken as the stable value. That is, the esophageal pressure at point 1 is determined. The processor 50 determines a pressure difference between an esophageal pressure, which corresponds to the target time point, and the stable value, as the endogenous positive end-expiratory pressure, that is, the esophageal pressure at point 1 is subtracted from the esophageal pressure at point 2, and the obtained difference is the endogenous positive end-expiratory pressure PEEPi. The processor 50 may also display, through a connected display, the calculated endogenous positive end-expiratory pressure PEEPi. Similarly, since the esophageal pressure data and the target parameter data can be obtained in real time, the endogenous positive end-expiratory pressure PEEPi can be calculated in real time, and the endogenous positive end-expiratory pressure PEEPi can be displayed in real-time and timely, which is helpful for a doctor to diagnose and treat the patient.

The method shown in FIG. 9 takes an inspiratory effort (pressure difference) which does not cause an increase in the gas flow rate as PEEPi. The calculation process is simple and reliable. The entire process does not require an expiratory hold operation, does not interrupt a normal ventilation process, and is suitable for us in both invasive and non-invasive ventilation.

In some embodiments, the medical device may include the above-mentioned processor, but not include the above-mentioned various sensors or not include some of the above-mentioned sensors, and some or all of the data obtained by sensors, such as an airway pressure, a gas flow rate, a carbon dioxide concentration, a chest impedance, etc., can be obtained by the processor from other devices. For example, the medical device may be a monitor, which obtains an airway pressure, a gas flow rate, a carbon dioxide concentration and other data from a ventilator or anesthesia machine, and obtains chest impedance and other data through an impedance sensor, thereby performing the above steps to monitor a positive end-expiratory pressure. For another example, the medical device can be other devices with data processing capabilities, such as various types of computer, central station, ultrasound imaging device etc., which obtain an airway pressure, a gas flow rate, a carbon dioxide concentration and other data from a ventilator or an anesthesia machine, and obtain chest impedance and other data, from a monitor, thereby performing the above steps to monitor a positive end-expiratory pressure.

Those skilled in the art can understand that all or part of the functions of various methods in the above embodiments can be implemented by hardware or by computer programs. When all or part of the functions in the above embodiments are implemented by a computer program, the program can be stored in a computer-readable storage medium. The storage medium can include a read-only memory, a random-access memory, a magnetic disk, an optical disk, a hard disk, etc., and the above functions can be achieved through execution of this program through a computer. For example, the program is stored in a device memory, and when the program in the memory is executed by the processor, all or part of the above functions can be realized. In addition, when all or part of the functions in the above embodiments are implemented by a computer program, the program can also be stored in a storage medium, such as a server, another computer, a magnetic disk, an optical disk, a flash disk or a mobile hard disk, and can be downloaded or copied to save it into the memory of the local device, or to perform a version update on the system of the local device. When the program in the memory is executed by the processor, all, or part of the functions in the above embodiments can be realized.

The description has been made with reference to various exemplary embodiments herein. However, those skilled in the art recognize that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified, or incorporated into other steps).

In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions, which are executed on a computer or other programmable data processing apparatus, can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

Although the principles herein have been shown in various embodiments, many modifications of structures, arrangements, proportions, elements, materials, and components particularly suitable for the specific environmental and operational requirements may be used without departing from the principles and scope of the present disclosure. The above modifications and other changes or amendments will be included in the scope of this disclosure.

The foregoing specific description has been described with reference to various embodiments. However, those skilled in the art will recognize that various modifications and changes can be made without departing from the scope of the present disclosure. Therefore, consideration of the present disclosure will be in an illustrative rather than a restrictive sense, and all such modifications will be included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "comprising", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communicational connection, functional connection, and/or any other connection.

It should be noted that a person of ordinary skill in the art could also make several variations and improvements without departing from the concept of this disclosure, and these variations and improvements would all fall within the scope of protection of this disclosure. Therefore, the protection scope of this disclosure should be in accordance with the appended claims.

**Claims**

1.  A method for measuring positive end-expiratory pressure, **characterized in that**, comprising:

    obtaining an airway pressure and a gas flow rate, when a medical ventilation device ventilates a patient; obtaining a ventilation volume according to the gas flow rate; and
    obtaining a total positive end-expiratory pressure, according to a preset respiratory mechanics equation, as well as the airway pressure, the gas flow rate, and the ventilation volume; wherein the respiratory mechanics equation is at least constructed from an airway pressure, a gas flow rate, a ventilation volume, and a total positive end-expiratory pressure.

2.  The method according to claim 1, **characterized in that**, further comprising:

    obtaining an exogenous positive end-expiratory pressure according to the airway pressure; and
    obtaining an endogenous positive end-expiratory pressure by subtracting the exogenous positive end-expiratory pressure from the total positive end-expiratory pressure.

3.  The method according to claim 1 or claim 2, **characterized in that**, the respiratory mechanics equation is at least constructed from an airway pressure, a pressure generated by respiratory muscle(s), a gas flow rate, a ventilation volume, and a total positive end-expiratory pressure.

4.  The method according to claim 1 or claim 2, **characterized in that**, the medical ventilation device ventilates the patient in a non-invasive ventilation mode.

5.  The method according to claim 4, **characterized in that**, further comprising:

    obtaining a gas leakage during non-invasive ventilation; and
    compensating the gas flow rate based on the gas leakage.

6.  The method according to claim 3, **characterized in that**, in the respiratory mechanics equation, the pressure generated by respiratory muscle(s) has a preset functional relationship with time.

7.  The method according to claim 6, **characterized in that**, the preset functional relationship comprises an exponential function, a trigonometric function, a piecewise function, or a polynomial function, or combination(s) thereof.

8.  The method according to claim 1, **characterized in that**, further comprising:
    obtaining, according to the total positive end-expiratory pressure, a recommended value, which is used by the medical ventilation device to set a positive end-expiratory pressure.

9.  The method according to claim 8, **characterized in that**, further comprising:

    displaying the recommended value;
    automatically setting the recommended value, so as to enable the medical ventilation device to ventilate the patient based on the recommended value; or
    receiving an instruction to set the recommended value as the positive end-expiratory pressure, and in response to the instruction, setting the recommended value, so as to enable the medical ventilation device to ventilate the patient based on the recommended value.

10. The method according to claim 2, **characterized in that**, further comprising:
    displaying at least one of the total positive end-expiratory pressure and the endogenous positive end-expiratory pressure.

11. The method according to claim 6, **characterized in that**, obtaining a total positive end-expiratory pressure, according to a preset respiratory mechanics equation, as well as the airway pressure, the gas flow rate, and the ventilation volume, comprises:

substituting multiple sets of airway pressures, gas flow rates and ventilation volumes at different time points into the preset respiratory mechanics equation, so as to obtain a set of equations for a pressure generated by respiratory muscle(s) and a total positive end-expiratory pressure; and
solving the set of equations, so as to obtain the total positive end-expiratory pressure.

12. The method according to claim 11, **characterized in that**, the respiratory mechanics equation comprises:

$$PEEPtot=k4*Paw+k1*Pmus-k2*Flow*R-k3*Volume*E;$$

wherein, Paw represents the airway pressure, Pmus represents the pressure generated by respiratory muscle(s), Flow represents the gas flow rate, Volume represents the ventilation volume, PEEPtot represents the total positive end-expiratory pressure, R represents a viscous resistance of a respiratory system, k1, k2, k3 and k4 represent preset empirical coefficients.

13. The method according to claim 12, **characterized in that**, when calculating the total positive end-expiratory pressure, the method further comprises:
for a patient without spontaneous respiration, setting the pressure generated by respiratory muscle(s) to be zero, or setting a corresponding preset empirical coefficient K1 to be zero.

14. A method for measuring endogenous positive end-expiratory pressure, **characterized in that**, comprising:

obtaining esophageal pressure data of a patient, when a medical ventilation device ventilates the patient; and
obtaining gas flow rate data, airway pressure data, concentration data of carbon dioxide, or chest impedance data, of the patient, when a medical ventilation device ventilates the patient;
obtaining a characteristic quantity, which characterizes that the patient overcomes an endogenous positive end-expiratory pressure, according to the gas flow rate data, the airway pressure data, the concentration data of carbon dioxide, or the chest impedance data; and
obtaining the endogenous positive end-expiratory pressure according to the esophageal pressure data and the characteristic quantity.

15. The method according to claim 14, **characterized in that**, obtaining the endogenous positive end-expiratory pressure according to the esophageal pressure data and the characteristic quantity, comprises:

obtaining a stable value of an esophageal pressure during an expiratory phase, according to the esophageal pressure data; and
determining a difference value between an esophageal pressure, which pressure corresponds to the characteristic quantity, and the stable value, as the endogenous positive end-expiratory pressure.

16. The method according to claim 14 or claim 15, **characterized in that**, the characteristic quantity is a target time point or a target time period.

17. The method according to claim 16, **characterized in that**, obtaining a characteristic quantity, which characterizes that the patient overcomes an endogenous positive end-expiratory pressure, according to the gas flow rate data, the airway pressure data, the concentration data of carbon dioxide, or the chest impedance data, comprises:
using a time point, when inhalation of the patient actually begins, as the target time point, wherein the time point is identified according to the gas flow rate data, the airway pressure data, the concentration data of carbon dioxide, or the chest impedance data.

18. The method according to claim 17, **characterized in that**:
using a time point, when inhalation of the patient actually begins, as the target time point, wherein the time point is identified according to the gas flow rate data, comprises:

using a time point, when a gas flow rate in an inspiratory period changes to a positive value, as the target time point, wherein said time point is identified according to the gas flow rate data;

using a time point, when inhalation of the patient actually begins, as the target time point, wherein the time point is identified according to the airway pressure data, comprises:

obtaining a minimum value of an airway pressure in an inspiratory period according to the airway pressure data, and using a time point which corresponds to the minimum value as the target time point;

using a time point, when inhalation of the patient actually begins, as the target time point, wherein the time point is identified according to the concentration data of carbon dioxide, comprises:

obtaining a time point, when a carbon dioxide concentration has a fastest decreasing rate, according to the concentration data of carbon dioxide, and using said time point as the target time point; or using a time point, when a carbon dioxide concentration is lower than a preset carbon dioxide concentration, as the target time point, according to the concentration data of carbon dioxide;

using a time point, when inhalation of the patient actually begins, as the target time point, wherein the time point is identified according to the chest impedance data, comprises:

obtaining waveform(s) of chest impedance according to the chest impedance data, obtaining a slope of each point of the waveform(s) of chest impedance, and using a time point, when a slope begins to be greater than a preset slope threshold, as the target time point.

19. A medical device, **characterized in that**, comprising:

a pressure sensor, which is configured to obtain an airway pressure of a patient during a ventilation process;
a flow sensor, which is configured to obtain a gas flow rate of the patient during the ventilation process;
a processor, which is configured to: obtain the airway pressure of the patient from the pressure sensor; obtain the gas flow rate of the patient from the flow sensor; obtain a ventilation volume according to the gas flow rate; and calculate to obtain a total positive end-expiratory pressure, according to a preset respiratory mechanics equation, as well as the airway pressure, the gas flow rate, and the ventilation volume; wherein the respiratory mechanics equation is at least constructed from an airway pressure, a gas flow rate, a ventilation volume, and a total positive end-expiratory pressure.

20. The medical device according to claim 19, **characterized in that**, the processor is further configured to:

obtain an exogenous positive end-expiratory pressure according to the airway pressure; and
obtain an endogenous positive end-expiratory pressure by subtracting the exogenous positive end-expiratory pressure from the total positive end-expiratory pressure.

21. The medical device according to claim 19 or claim 20, **characterized in that**, the respiratory mechanics equation is at least constructed from an airway pressure, a pressure generated by respiratory muscle(s), a gas flow rate, a ventilation volume, and a total positive end-expiratory pressure.

22. The medical device according to claim 19 or claim 20, **characterized in that**, the medical ventilation device ventilates the patient in a non-invasive ventilation mode.

23. The medical device according to claim 22, **characterized in that**, the processor is further configured to:

obtain a gas leakage during non-invasive ventilation; and
compensate the gas flow rate based on the gas leakage.

24. The medical device according to claim 21, **characterized in that**, in the respiratory mechanics equation, the pressure generated by respiratory muscle(s) has a preset functional relationship with time.

25. The medical device according to claim 24, **characterized in that**, the preset functional relationship comprises an exponential function, a trigonometric function, a piecewise function, or a polynomial function, or combination(s) thereof.

26. The medical device according to claim 19, **characterized in that**, the processor is further configured to:
obtain, according to the total positive end-expiratory pressure, a recommended value, which is used by the medical ventilation device to set a positive end-expiratory pressure.

27. The medical device according to claim 26, **characterized in that**, the processor is further configured to:

> display, through a connected display, the recommended value;
> automatically set the recommended value, so as to enable a respiratory support system to ventilate the patient based on the recommended value; or
> receive an instruction to set the recommended value as the positive end-expiratory pressure, and in response to the instruction, set the recommended value, so as to enable a respiratory support system to ventilate the patient based on the recommended value.

28. The medical device according to claim 20, **characterized in that**, the processor is further configured to:
display, through a connected display, at least one of the total positive end-expiratory pressure and the endogenous positive end-expiratory pressure.

29. The medical device according to claim 24, **characterized in that**, in order to obtain a total positive end-expiratory pressure, according to a preset respiratory mechanics equation, as well as the airway pressure, the gas flow rate, and the ventilation volume, the processor is further configured to:

> substitute multiple sets of airway pressures, gas flow rates and ventilation volumes at different time points into the preset respiratory mechanics equation, so as to obtain a set of equations for a pressure generated by respiratory muscle(s) and a total positive end-expiratory pressure; and
> solve the set of equations, so as to obtain the total positive end-expiratory pressure.

30. The medical device according to claim 29, **characterized in that**, the respiratory mechanics equation comprises:

$$PEEPtot = k4*Paw + k1*Pmus - k2*Flow*R - k3*Volume*E;$$

> wherein, Paw represents the airway pressure, Pmus represents the pressure generated by respiratory muscle(s), Flow represents the gas flow rate, Volume represents the ventilation volume, PEEPtot represents the total positive end-expiratory pressure, R represents a viscous resistance of a respiratory system, k1, k2, k3 and k4 represent preset empirical coefficients.

31. The medical device according to claim 30, **characterized in that**, in order to calculate the total positive end-expiratory pressure, the processor is further configured to:
for a patient without spontaneous respiration, set the pressure generated by respiratory muscle(s) to be zero, or set a corresponding preset empirical coefficient K1 to be zero.

32. A medical device comprising a processor, wherein the medical device further comprises a pressure sensor, a flow sensor, a carbon dioxide sensor, or an impedance sensor, **characterized in that**;

> the pressure sensor is configured to obtain an airway pressure of a patient during a ventilation process;
> the flow sensor is configured to obtain a gas flow rate of the patient during a ventilation process;
> the carbon dioxide sensor is configured to obtain a carbon dioxide concentration of the patient during a ventilation process;
> the impedance sensor is configured to obtain chest impedance of the patient during a ventilation process;
> the processor is configured to:
>
>> obtain esophageal pressure data of the patient, when a respiratory support system ventilates the patient; and obtain gas flow rate data, airway pressure data, concentration data of carbon dioxide, or chest impedance data, of the patient, when a respiratory support system ventilates the patient;
>> obtain a characteristic quantity, which characterizes that the patient overcomes an endogenous positive end-expiratory pressure, according to the gas flow rate data, the airway pressure data, the concentration data of carbon dioxide, or the chest impedance data; and
>> obtain the endogenous positive end-expiratory pressure according to the esophageal pressure data and the characteristic quantity.

33. The medical device according to claim 32, **characterized in that**, in order to obtain the endogenous positive end-expiratory pressure according to the esophageal pressure data and the characteristic quantity, the processor is further configured to:

obtain a stable value of an esophageal pressure during an expiratory phase, according to the esophageal pressure data; and

determine a difference value between an esophageal pressure, which pressure corresponds to the characteristic quantity, and the stable value, as the endogenous positive end-expiratory pressure.

**34.** The medical device according to claim 32 or claim 33, **characterized in that**, the characteristic quantity is a target time point or a target time period.

**35.** The medical device according to claim 34, **characterized in that**, in order to obtain a characteristic quantity, which characterizes that the patient overcomes an endogenous positive end-expiratory pressure, according to the gas flow rate data, the airway pressure data, the concentration data of carbon dioxide, or the chest impedance data, the processor is further configured to:

use a time point, when inhalation of the patient actually begins, as the target time point, wherein the time point is identified according to the gas flow rate data, the airway pressure data, the concentration data of carbon dioxide, or the chest impedance data.

**36.** The medical device according to claim 35, **characterized in that**:

in order to use a time point, when inhalation of the patient actually begins, as the target time point, wherein the time point is identified according to the gas flow rate data, the processor is further configured to:

use a time point, when a gas flow rate in an inspiratory period changes to a positive value, as the target time point, wherein said time point is identified according to the gas flow rate data;

in order to use a time point, when inhalation of the patient actually begins, as the target time point, wherein the time point is identified according to the airway pressure data, the processor is further configured to:

obtain a minimum value of an airway pressure in an inspiratory period according to the airway pressure data, and use a time point which corresponds to the minimum value as the target time point;

in order to use a time point, when inhalation of the patient actually begins, as the target time point, wherein the time point is identified according to the concentration data of carbon dioxide, the processor is further configured to:

obtain a time point, when a carbon dioxide concentration has a fastest decreasing rate, according to the concentration data of carbon dioxide, and use said time point, as the target time point; or use a time point, when a carbon dioxide concentration is lower than a preset carbon dioxide concentration, as the target time point, according to the concentration data of carbon dioxide;

in order to use a time point, when inhalation of the patient actually begins, as the target time point, wherein the time point is identified according to the chest impedance data, the processor is further configured to:

obtain waveform(s) of chest impedance according to the chest impedance data, obtain a slope of each point of the waveform(s) of chest impedance, and use a time point, when a slope begins to be greater than a preset slope threshold, as the target time point.

**37.** A medical device, **characterized in that**, comprising:

a memory, which is configured to store program(s); and
a processor, which is configured to implement the method according to any one of claims 1-18 by executing program(s) which is(are) stored in the memory.

**38.** A computer-readable storage medium, **characterized in that**, comprising program(s), which is(are) capable of being executed, so as to implement the method according to any one of claims 1-18.

Pressure sensor
10

Flow sensor   30

Processor
50

FIG. 1

Pressure sensor
10

Processor
50

FIG. 2

Carbon dioxide
sensor 20

Processor
50

FIG. 3

Processor
50

Flow sensor   30

FIG. 4

FIG. 5

Expiratory
branch 213a

213c

Patient

211

214a

Inspiratory
branch 213b

214b

212

Gas

Memory
215

Processor 50

Display 70

FIG. 6

Expiratory branch 340a

Patient

311

340c

Inspiratory branch
340b

Anesth
etic
output
apparat
us

330

Respirato
ry
assistance
apparatus

320

312

G
A
S

Memory
350

Processor 50

Display 70

FIG. 7

Obtaining an airway pressure and a gas flow rate, when a medical ventilation device ventilates a patient

1

Obtaining a ventilation volume according to the gas flow rate

2

obtaining a total positive end-expiratory pressure, according to a preset respiratory mechanics equation, as well as the airway pressure, the gas flow rate, and the ventilation volume

3

Obtaining an exogenous positive end-expiratory pressure according to the airway pressure

4

Obtaining an endogenous positive end-expiratory pressure by subtracting the exogenous positive end-expiratory pressure from the total positive end-expiratory pressure

5

FIG. 8

Obtaining esophageal pressure data of a patient, when a medical ventilation device ventilates the patient; and obtaining gas flow rate data, airway pressure data, concentration data of carbon dioxide, or chest impedance data, of the patient, when a medical ventilation device ventilates the patient

1'

Obtaining a characteristic quantity, which characterizes that the patient overcomes an endogenous positive end-expiratory pressure, according to the gas flow rate data, the airway pressure data, the concentration data of carbon dioxide or the chest impedance data

2'

Obtaining the endogenous positive end-expiratory pressure according to the esophageal pressure data and the characteristic quantity

3'

FIG. 9

FIG. 10

FIG. 11

### INTERNATIONAL SEARCH REPORT

| | International application No. |
|---|---|
| | **PCT/CN2022/103904** |

| A. CLASSIFICATION OF SUBJECT MATTER |
|---|
| A61M 16/00(2006.01)i; A61B 5/08(2006.01)i |
| According to International Patent Classification (IPC) or to both national classification and IPC |

| B. FIELDS SEARCHED |
|---|
| Minimum documentation searched (classification system followed by classification symbols) |
| A61M, A61B |
| Documentation searched other than minimum documentation to the extent that such documents are included in the fields searched |
| Electronic data base consulted during the international search (name of data base and, where practicable, search terms used) |
| CNABS, CNTXT, VEN, OETXT: 呼气末, 气道压力, 流速, 流量, 流率, 模型, 方程, 食道, 食管, PEEPi, pressure, airway, trach +, esophag+, gullet+ |

| C. DOCUMENTS CONSIDERED TO BE RELEVANT |
|---|

| Category* | Citation of document, with indication, where appropriate, of the relevant passages | Relevant to claim No. |
|---|---|---|
| X | US 2021016035 A1 (CONVERGENT ENG INC) 21 January 2021 (2021-01-21) description, paragraphs [0052]-[0138], and figures 1-17 | 14-18, 32-38 |
| Y | CN 110545872 A (KONINKL PHILIPS NV) 06 December 2019 (2019-12-06) description, paragraphs [0020]-[0070], and figures 1-8 | 1-13, 19-31, 37, 38 |
| Y | "基于主动模拟肺的通气模式关键技术研究 (Research on Key Technologies of Ventilation Mode Based on Active Servo Lung)" 国防科学技术大学博士学位论文 *(Non-official translation: Doctoral Dissertation of National University of Defense Technology)*, 01 June 2009 (2009-06-01), pp. 51-64 | 1-13, 19-31, 37, 38 |
| A | CN 103182120 A (BEIJING AEONMED CO., LTD.) 03 July 2013 (2013-07-03) entire document | 1-38 |
| A | CN 111543999 A (LOWENSTEIN MEDICAL TECHNOLOGY GMBH & CO. KG) 18 August 2020 (2020-08-18) entire document | 1-38 |
| A | US 2014171817 A1 (BLANCH PAUL B et al.) 19 June 2014 (2014-06-19) entire document | 1-38 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **22 September 2022** | **29 September 2022** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/103904** |

| C. | DOCUMENTS CONSIDERED TO BE RELEVANT | |
|---|---|---|
| Category* | Citation of document, with indication, where appropriate, of the relevant passages | Relevant to claim No. |
| A | US 2005284476 A1 (BLANCH PAUL B et al.) 29 December 2005 (2005-12-29)<br>entire document | 1-38 |

Form PCT/ISA/210 (second sheet) (January 2015)

**INTERNATIONAL SEARCH REPORT**

| International application No. |
|---|
| **PCT/CN2022/103904** |

**Box No. III    Observations where unity of invention is lacking (Continuation of item 3 of first sheet)**

This International Searching Authority found multiple inventions in this international application, as follows:

[1]   Claims 1-13 set forth a method for detecting the positive end-expiratory pressure. Claims 14-18 set forth a method for detecting the intrinsic positive end-expiratory pressure. Claims 19-31 set forth a medical device for detecting the positive end-expiratory pressure. Claims 32-36 set forth a medical device for detecting and detecting the intrinsic positive end-expiratory pressure. Independent claim 1 or 19 and independent claim 14 or 32 do not share a same or corresponding technical feature, do not belong to a single general inventive concept, and therefore do not satisfy the requirement of unity of invention and do not comply with PCT Rule 13.1.

1. ☐   As all required additional search fees were timely paid by the applicant, this international search report covers all searchable claims.

2. ☑   As all searchable claims could be searched without effort justifying additional fees, this Authority did not invite payment of additional fees.

3. ☐   As only some of the required additional search fees were timely paid by the applicant, this international search report covers only those claims for which fees were paid, specifically claims Nos.:

4. ☐   No required additional search fees were timely paid by the applicant. Consequently, this international search report is restricted to the invention first mentioned in the claims; it is covered by claims Nos.:

**Remark on Protest**   ☐   The additional search fees were accompanied by the applicant's protest and, where applicable, the payment of a protest fee.

☐   The additional search fees were accompanied by the applicant's protest but the applicable protest fee was not paid within the time limit specified in the invitation.

☐   No protest accompanied the payment of additional search fees.

Form PCT/ISA/210 (continuation of first sheet) (January 2015)

**INTERNATIONAL SEARCH REPORT**
**Information on patent family members**

International application No.

**PCT/CN2022/103904**

| Patent document cited in search report | | | Publication date (day/month/year) | Patent family member(s) | | | Publication date (day/month/year) |
|---|---|---|---|---|---|---|---|
| US | 2021016035 | A1 | 21 January 2021 | WO | 2021011748 | A1 | 21 January 2021 |
| | | | | EP | 3998940 | A1 | 25 May 2022 |
| CN | 110545872 | A | 06 December 2019 | US | 2019374733 | A1 | 12 December 2019 |
| | | | | WO | 2018153964 | A1 | 30 August 2018 |
| | | | | JP | 2020508194 | A | 19 March 2020 |
| | | | | EP | 3585465 | A1 | 01 January 2020 |
| CN | 103182120 | A | 03 July 2013 | MX | 2013011849 | A | 01 November 2013 |
| | | | | BR | 112013026139 | A2 | 19 January 2021 |
| | | | | US | 2014053840 | A1 | 27 February 2014 |
| | | | | WO | 2013097697 | A1 | 04 July 2013 |
| CN | 111543999 | A | 18 August 2020 | | None | | |
| US | 2014171817 | A1 | 19 June 2014 | | None | | |
| US | 2005284476 | A1 | 29 December 2005 | WO | 2006012205 | A2 | 02 February 2006 |
| | | | | US | 2009293877 | A1 | 03 December 2009 |
| | | | | JP | 2008504068 | A | 14 February 2008 |
| | | | | EP | 1765442 | A2 | 28 March 2007 |

Form PCT/ISA/210 (patent family annex) (January 2015)